(19) Europäisches Patentamt
European Patent Office
Office européen des brevets

(11) Publication number: **0 329 361 B1**

# (12) EUROPEAN PATENT SPECIFICATION

(45) Date of publication of patent specification: **06.12.95**

(51) Int. Cl.[6]: **C07F 9/117**, C12P 17/06, //(C12P17/06,C12R1:585)

(21) Application number: **89301336.7**

(22) Date of filing: **13.02.89**

## (54) 2-pyranone derivatives and process for production thereof.

(30) Priority: **13.02.88 JP 30069/88**

(43) Date of publication of application: **23.08.89 Bulletin 89/34**

(45) Publication of the grant of the patent: **06.12.95 Bulletin 95/49**

(84) Designated Contracting States: **AT DE ES FR GB IT SE**

(56) References cited:
**EP-A- 0 087 021**
**EP-A- 0 157 553**

(73) Proprietor: **SUNTORY LIMITED**
**1-40, Dojimahama 2-chome**
**Kita-ku,**
**Osaka-shi,**
**Osaka-fu 530 (JP)**

(72) Inventor: **Maeda, Mitsuru**
**2-15-5, Kinugawa**
**Ohtsu-shi**
**Shiga (JP)**
Inventor: **Kodama, Tohru**
**2-3-11, Higashikanmaki**
**Takatsuki-shi**
**Osaka (JP)**
Inventor: **Asami, Sumio**
**16-16, Futaba-cho**
**Ibaraki-shi**
**Osaka (JP)**
Inventor: **Amano, Norihide**
**7-17, Kitasonomachi**
**Takatsuki-shi**
**Osaka (JP)**
Inventor: **Kusumi, Takaaki**
**2-12-21-402, Yamate-cho**
**Suita-shi**
**Osaka (JP)**
Inventor: **Hosono, Hidekazu**
**1177-2, Ichigao-cho**
**Midori-ku**
**Yokohama-shi**
**Kanagawa (JP)**

(74) Representative: **Harrison, David Christopher et al**
**MEWBURN ELLIS**
**York House**
**23 Kingsway**
**London WC2B 6HP (GB)**

EP 0 329 361 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel 2-pyranone derivatives, processes for production thereof, and to antimicrobial compositions containing them. In particular, the derivatives may exhibit antimicrobial activity at a very low concentration, especially against plant pathogenic fungi and therefore are useful as an active ingredient for a agricultural biocidal composition.

The problem addressed is the provision of new biocidal compounds which do not exhibit disadvantageous side effects on agricultural plants, and may be useful in particular for prevention and control of Botrytis cinerea which can be parasitic on a very wide range of plants (for example cucumber, tomato, eggplant, strawberry, lettuce, udo (Aralia cordata), and onion), at low temperature and high moisture condition.

In addressing this problem the present inventors worked to isolate from nature a microorganism which produces biologically active compounds suppressing gray mold, and found that an actinomyces belonging to the genus Streptomyces produces in a culture broth substances which exhibit a growth-preventing effect on fungi including dermatophytes and plant pathogenic fungi at a very low concentration. The present inventors further isolated and purified the substance and determined a structure thereof, and confirmed that the purified substance strongly suppresses gray mold.

Accordingly, the present invention provides a 2-pyranone derivative represented by the formula (I):

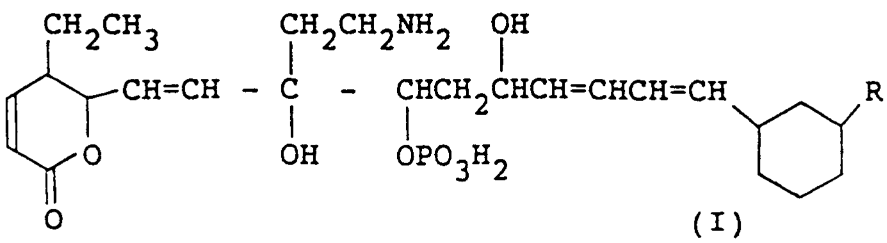

(I)

wherein R represents a hydrogen atom, or a linear or branched alkyl carbonyloxy, or cyclo-alkyl carbonyloxy group having 3 to 10 carbon atoms, and salts thereof.

In a second aspect there is provided a process for the production of a 2-pyranone derivative (I) as defined, comprising the steps of culturing a microorganism belonging to the strain Streptomyces platensis SAM-0654 (FERM BP-1668), capable of producing the derivative, to produce the derivative, and recovering the produced derivative from the cultured product.

In a further aspect there is provided a biocidal composition comprising a biocidally effective amount of the above-mentioned 2-pyranone derivative. Still further, the present invention provides said microorganism strain capable of producing the above-mentioned 2-pyranone derivatives.

US-A-4578383, which corresponds to EP-A-87021, and EP-A-128651, describe a compound having a basic structure of the formula (I) above but having a hydrogen atom or a hydroxyl group at the 5-position of the pyranone ring, rather than the ethyl group in formula (I), and having a 1-propenyl or 3-hydroxy-1-propenyl at a position most distant from the pyranone ring, rather than a cyclohexyl ring as in formula (I). Therefore these compounds are significantly different in structure from the present compounds which have an ethyl group at the 5-position of the pyranone ring and a cyclohexyl ring structure at the position most distant from the pyranone structure. Moreover, the above-mentioned publications do not suggest that a microorganism belonging to Streptomyces might produce the compounds.

US-A-4575500 discloses a compound having a structure similar to that of formula (I) except for a cyclohexenone structure instead of the pyranone ring of the present invention.

Figure 1 represents an elution profile where components in a concentrated supernatant from a culture broth were separated by a high performance liquid chromatography.

Preferred features of the invention, and specific examples of embodiments, are now described.

In the above-mentioned formula (I), the alkylcarbonyloxy group is preferably butyryloxy, isobutyryloxy, isovaleryloxy, 2-methylbutyryloxy, cyclohexylcarbonyloxy, 4-methylhexanoyloxy, 6-methyl-heptanoyloxy, or octanoyloxy.

The producer microorganism may be any microorganism belonging to the genus Streptomyces and capable of producing the present 2-pyranone derivatives.

Such a microorganisms may be obtained by isolating microorganisms belonging to Streptomyces from microbial sources such as soil, by a conventional procedure for the isolation of actinomyces, and selecting a microorganism which produces a substance having an antimicrobial activity against Botrytis cinerea. A

producer strain embodying the invention thus obtained, Streptomyces platensis SAM-0654, was deposited with the Fermentation Research Institute, Agency of Industrial Science and Technology on January 22, 1988, as FERM BP-1668.

The strain SAM-0654 has the following taxonomical properties.

1. Morphology

An aerial mycelium shows simple branches, and at the top thereof, a spiral spore chain comprising 10 to 50 or more spores is formed. The spore is semispherical (crescent), has a length of 0.6 to 1.0 μm and a width of 0.3 to 0.4 μm, and has a smooth surface.

2. Cultural characteristics on various media

The appearance of a culture on various media is set forth in Table 1. The observation was carried out after culturing at 28°C for 21 days.

Table 1

| Culture media | Growth | Aerial mycelium | | Vegetative mycelium | Soluble pigment | Other appearance |
|---|---|---|---|---|---|---|
| Yeast extract - Malt extract Agar (ISP-2) | Good | Abundant | White - Light yellowish - Gray | Brown | Yellow | Hygroscopic |
| Oat meal agar (ISP-3) | Good | Abundant | White - Gray | Yellowish brown - Brown | Yellow | Hygroscopic |
| Inorganic salts - Starch agar (ISP-4) | Good | Abundant | White - Light yellowish - Gray | Yellow - Yellowish brown | Light yellow | Hygroscopic |
| Glycerol - Asparagine agar (ISP-5) | Good | Abundant | White - Light yellowish gray | Orange - Yellowish brown | Yellow | |
| 1/10 V-8 agar | Good | Moderate | White - Gray | Deep greenish gray | — | Hygroscopic |
| Yeast extract - Starch agar | Good | Abundant | White | Yellow - Light yellowish brown | Light yellow | No spore formation |
| 1/10 Potato - Carrot agar | Good | Moderate | White - Light gray | White - Light yellow | — | Hygroscopic |
| Calcium malate agar (+ Glycerol) | Good | Poor | White | Yellow | — | |

3. Physiological properties

| | | |
|---|---|---|
| (1) | Scope of growth temperature | 20°C to 37° |
| (2) | Liquification of gelatine | negative |
| (3) | Hydrolysis of starch | positive |
| (4) | Coagulation of skim milk (37°C) | negative |
| (5) | Peptonization of skim milk (37°C) | negative |
| (6) | Production of melanoid pigments | |
| | Tryptone-yeast extract agar medium | negative |
| | Tyrosine agar medium | negative |
| | Peptone-yeast extract-iron agar medium | negative |
| (7) | Nitrate reduction | negative |
| (8) | Utilization of carbon sources | |

| | | | |
|---|---|---|---|
| L-arabinose | - | L-rhamnose | - |
| D-xylose | ± | raffinose | + |
| D-glucose | ++ | D-mannitol | ++ |
| D-fructose | + | lactose | - |
| sucrose | ++ | salicin | - |
| inositol | ++ | cellulose | - |

4. Chemotaxonomical properties

(1) 2,6-diaminopimelic acid

Whole-cell hydrolyzates of SAM 0654 contained L,L-2,6-diaminopimelic acid.

(2) Quinone system

The strain SAM 0654 has melaquinone-9(H6) and melaquinone -9(H8) as major quinones.

From the above-mentioned taxonomical properties, the present strain SAM-0654 is considered to be an actinomyces belonging to the genus Streptomyces. Comparison of the present strain with the known species of the genus Streptomyces, on the basis of the above-mentioned taxonomical properties, revealed that the present strain is most similar to Streptomyces platensis (International Journal of Systematic Bacteriology, Vol. 18, pp 360, 1968).

Accordingly, the present strain SAM-0654 was compared with a type strain of Streptomyces platensis, viz. Streptomyces platensis JCM 4662, and a summary of the results thereof is given in Table 2.

Table 2

| | | | SAM0654 | Streptomyces platensis JCM 4662 |
|---|---|---|---|---|
| Spore chain | | | Spiral | Spiral |
| Surface and shape of spore | | | Smooth, subspherical (crescent) | Smooth, subspherical (crescent) |
| Cultural charac-teristics | Yeast extract - Malt extract agar (ISP-2) | Aerial mycelium | White - Gray | Gray |
| | | Vegetative mycelium | Brown | Reddish brown |
| | | Soluble pigments | Yellow | Light brown |
| | | Other appearance | Hygroscopic | Hygroscopic |
| | Oat meal agar (ISP-3) | Aerial mycelium | White - Gray | Gray |
| | | Vegetative mycelium | Yellowish brown | Reddish brown |
| | | Soluble pigments | Yellow | Light brown |
| | | Other appearance | Hygroscopic | Hygroscopic |
| | Inorganic salt - Starch agar (ISP-4) | Aerial mycelium | White - Gray | Gray |
| | | Vegetative mycelium | Light yellowish brown | Reddish brown |
| | | Soluble pigments | Light yellow | Light brown |
| | | Other appearance | Hygroscopic | Hygroscopic |
| | Glycerol - asparagine agar (ISP-5) | Aerial mycelium | Light yellowish gray | Gray |
| | | Vegetative mycelium | Yellowish brown | Yellowish brown |
| | | Soluble pigments | Yellow | ____ |
| | | Other appearance | ____ | ____ |

Table 2 (continued)

| | SAM0654 | Streptomyces platensis JCM 4662 |
|---|---|---|
| Production of melanoid pigments | negative | negative |
| Liquification of gelatine | negative | negative |
| Hydrolysis of starch | positive | positive |
| Coagulation and peptonization of skim milk | negative | negative |
| Utilization of carbon source | Glucose, fructose, sucrose, inositol, raffinose, mannitol | Glucose, fructose, sucrose, inositol, raffinose, mannitol |

As seen from Table 2, properties of the SAM-0654 are very similar to those of Streptomyces platensis, and the only difference is that color tone of the vegetative mycelium and soluble pigments. In the type strain of Streptomyces platensis, Streptomyces platensis JCM 4662, the color tone of the vegetative mycelium and soluble pigments is reddish; an the other hand in the strain SAM-0654 it is yellowish. Nevertheless, the present inventors consider these differences to be not enough to separate the strain SAM-0654 from Streptomyces platensis, and then identified the strain SAM-0654 as Streptomyces platensis SAM 0654.

For a production of the present compound, a producer strain is preferably cultured in a liquid medium under an aerobic condition provided by shaking or by aeration and agitation, although a solid medium may

be used. Any medium in which a producer microorganism can grow and produce the present compound may be used, as long as the medium contains a carbon source such as glucose, lactose, glycerol, starch, sucrose, dextrin, molasses and/or organic acids, and a nitrogen source, such as hydrolyzed protein product such as peptone or casamino acid, meat extract, yeast extract, defatted soybean pellet, corn steep liquor, amino acids, ammonium salts, nitrate or other organic or inorganic nitrogen-containing substances. Inorganic salts, such as various phosphates, magnesium sulfate, and/or sodium chloride may be added to a medium. Moreover, vitamins and nuoleic acid-related compounds may be added to accelerate the growth of the strain. Note, in some cases, the addition of an antifoamer such as silicone, propylene glycol derivatives, and soy bean oil may increase the accumulation level of the present compound.

When culturing, preferably a preculture is prepared on a small scale, and the preculture is inoculated to a production culture medium, although direct inoculation to a production culture medium may be allowed. Culture conditions including culture temperature, pH value of culture medium, and duration of culture are chosen or controlled so that the conditions provide a maximum production of the present compound. Usually, culturing is carried out at 25°C to 35°C, at pH 5.5 - 7.2, under an aerobic condition, for two to three days.

During the culturing, the present compounds are extracellularly accumulated in a culture broth. Therefore, in a preferred embodiment for recovering the products, a culture broth is filtrated or centrifuged to obtain a filtrate or supernatant containing the products, and the desired products are recovered from the filtrate or supernatant. Alternatively, the product can be directly recovered from a culture broth. The recovery process is followed by a disk method using Aspergillus oryzae or Botrytis cinerea as a test organism, or a cucumber seedling assay described in the examples.

The desired compound is isolated and purified from the culture broth by various procedures chosen according to the nature of the desired compound. Namely, extraction using a organic solvent, such as 1-butanol, which is immiscible with water and dissolves the desired compound, dissolution in a high polar solvent such as methanol or ethanol, removal of impurities by treatment with hexane or the like, gel filtration through Sephadex, ion exchange chromatography on an ion exchange resin, Sephadex ion exchanger or the like, or adsorption chromatography on active carbon, silica gel or Amberlite XAD-1 or -2, or a combination thereof, may be used to isolate and purify the desired compound. As especially preferable adsorbents, Diaion HP-20 (Mitsubishi Chemical Industry Ltd.), Sepabeads FP-DA13 (Mitsubishi Chemical Industry Ltd.), YMC-$C_{18}$ (Yamamura Chemical Laboratories Co. Ltd.), and a combination thereof, are mentioned. Note, since a culture broth from the producer strain contains more than ten analogues, separation and purification of each active component can be most effectively carried out by high performance liquid chromatography.

Figure 1 represents a chromatogram of separation of components shown by absorbance at 232 nm, obtained using a column YMC-$C_{18}$ (50 mm of an inner diameter and 300 mm of a length, Yamamura Chemical Laboratories Co. Ltd.) and a gradient elution with 40% to 60% acetonitrile containing 0.1% formic acid at a flow rate of 32 ml/minute. Each peak was fractionated, the component was purified, and each component was found to have the following structure (I):

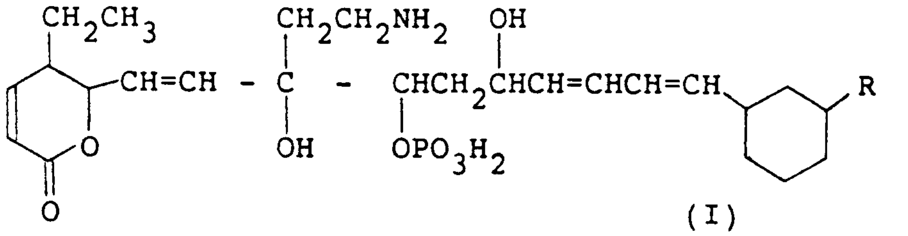

(I)

Referring to the above-mentioned general formula and Fig. 1;

Peak A in Fig. 1 represents a compound (I-a) embodying the invention, wherein R in the formula (I) is a hydrogen atom;

Peak B represents a mixture of a compound (I-b), wherein R is n-butyryloxy, and a compound (I-c), wherein R is isobutyryloxy;

Peak C represents a mixture of a compound (I-d), wherein R is isovaleryloxy, and a compound (I-e), wherein R is 2-methylbutyryloxy;

Peak E represents a compound (I-f), wherein R is cyclohexylcarbonyloxy;

Peak F represents a compound (I-g), wherein R is 4-methylhexanoyloxy;

Peak H represents a compound (I-h), wherein R is 6-methylheptanoyloxy; and

Peak I represents a mixture of a major compound (I-i), wherein R is octanoyloxy, and a compound (I-j), wherein R is $C_8H_{15}COO$-.

In addition to the above-mentioned compounds, it was found that minor compounds wherein R is, for example, propionyloxy, valeryloxy, or 4-methyl-valeryloxy, are present, on the basis of NMR spectra and Mass spectra.

The present compounds can be present in the form of salts, such as hydrochloride, phosphate, nitrate.

The above-mentioned compounds embodying the invention suppress the gray mold at low concentration.

When the present compound is used as a antimicrobial agent, it may be admixed with a carrier, and if necessary, with other additives to form a conventional formulation used as an agricultural antimicrobial agent. This may be solid e.g. finely divided powders or granule material, or liquid, e.g. solution, emulsion, suspension, concentrate, slurry or the like. Suitable liquid carriers include water; alcohols such as ethanol, ethyleneglycol; ketones such as acetone; ethers such as dioxane, cellosolves; aliphatic hydrocarbons such as kerosene; aromatic hydrocarbons such as benzene, toluene; organic bases such as pyridine; halogenated hydrocarbons such as chloroform, carbon tetrachloride; esters such as ethyl acetate, fatty acid glycerol esters; nitriles such as acetonitrile; dimethylformamide; and dimethyl sulfoxide.

Suitable solid carriers include powders having a plant origin, such as starches, wheat flour; and mineral powders such as kaolin, bentonite, calcium phosphate, clays, talks, silicas. These can be used alone or in combination.

Moreover, as the emulsifier, fixing agent, penetrating agent or dispersant, soap, sulfate esters of higher alcohols, alkyl sulfonic acid, alkyl aryl sulfonic acids, tertiary ammonium salts, oxyalkylamines, fatty acid esters, polyalkyleneoxides, and anhydrosolbitols are used. Usually, these additives are used in an amount of 0.2 to 10% by weight relative to the formulation. Further, other antimicrobial agents, insecticides, nematocides, herbicides, plant growth regulators, plant nutrients, fertilizer, and/or soil modifiers can be included in the formulation.

Antimicrobial compositions of the present invention may be produced by conventional procedures from the 2-pyranone derivative (I), carriers, additives and the like.

The present compound (I) is preferably contained in an amount of 0.5 to 50% by weight in a solid formulation, and in an amount of 5 to 90% by weight in a liquid formulation. Note, the liquid formulations are preferably diluted by an appropriate amount of water, for example, 50 to 5000-fold, prior to application.

An amount of the present compound (I) used, and the ratio thereof to other components in the composition, depends on the stage of growth of a plant to which the composition is to be applied, condition of growth of the plant, a nature of the pathogen, condition of pathology, method of application and the like, and is usually 10 to 300 g of the present compound (I) per 10 ares. A concentration of the compound (I) in a liquid composition in use is usually 10 to 1000 ppm. The method of application of the composition is not particularly limited, and it can be applied by, for example, spraying, dusting or by irrigation, or in the form of a seed coat, as long as it is applied safely and effectively to a target plant.

## Examples

The present invention will be further illustrated by, but is by no means limited to, the following examples.

### Example 1. Production of compounds

First, 30 ℓ of a medium containing 90 g glucose, 300 g peptone, 600 g corn starch, 60 g yeast extract, 90 g dry yeast cells, and 30 g dipotasium phosphate (pH 7.0) was inoculated with a pure culture of Streptomyces platensis SAM-0654 (FERM BP-1668), and culturing was carried out in a small fermentor at 28 °C, with aeration at 30 ℓ/minute and agitation at 400 rpm, for 40 hours.

The culture broth thus obtained was centrifuged to obtain 27 ℓ of a supernatant, which was then passed through a column (14 cm x 33 cm) packed with Diaion HP-20 (Mitsubishi Chemical Industry Ltd.) to adsorb the product. The column was washed with 30 ℓ of water and 36 ℓ of 50% methanol, and was eluted with 18 ℓ of methanol to obtain a eluate having an anti-Aspergillus (or Botrytis) activity. The eluate was concentrated under a reduced pressure, centrifuged to eliminate impurities, and 400 ml of the concentrate was applied to a column (5.6 cm x 33 cm) packed with Sepabeads FP-DA13 (Mitsubishi Chemical Industry Ltd.), which had been washed with methanol, and the column was eluted with methanol. Since an anti-microbial activity was found at an elution volume of 3000 ml to 6600 ml, this fraction was concentrated under a reduced pressure, water was added to the concentrate, and the mixture was lyophilized to obtain 800 mg of

a dry preparation. Then 100 mg of the dry preparation was dissolved in methanol, and the solution was applied to a high performance liquid chromatography column (YMC $C_{18}$ column, having an inner diameter of 50 mm and a length of 300 mm; Yamamura Chemical Laboratories Co. Ltd.). Elution was carried out with 40% acetonitrile in 0.1% formic acid for 8 minutes, a linear gradient of 40% to 60% acetonitrile in 0.1% formic acid for 60 minutes, and 60% acetonitrile in 0.1% formic acid for 10 minutes, at a flow rate of 32 ml/minute, while monitoring by absorbance of 232 nm, and 25.6 ml (0.8 minute) fractions were obtained. This procedure was repeated eight times.
The chromatogram thereof is shown in Fig. 1.

Fractions No. 31 to 33 containing a peak A provided 13 mg of the above-mentioned compound I-a;

fractions No. 35 to 37 containing a peak B provided 50 mg of a mixture comprising the compound I-b and the compound I-c (1:2);

fractions No. 44 to 47 containing a peak C provided 120 mg of a mixture comprising the compound I-d and the compound I-e (3:2);

fractions No. 60 to 63 containing a peak E provided 119 mg of the compound I-f;

fractions No. 67 to 69 containing a peak F provided 35 mg of the compound I-g; and

fractions No. 81 to 87 containing peaks H and I provided 84 mg of a mixture comprising the compound I-h, the compound I-i, and the compound I-j (3:2:1).

Further, the mixture comprising the compounds I-a and I-c, the mixture comprising the compounds I-d and I-e, and the mixture comprising the compounds I-h, I-i, and I-j were separately subjected to high performance liquid chromatography under the same conditions as described above, and as a result, the compounds I-b and I-c, the compounds I-d and I-e, and the compounds I-h, I-i and I-j were isolated, respectively.

The compounds I-a to I-j thus obtained were white powders, exhibited a maximum ultraviolet absorbance at 232 nm in methanol, had a positive ninhydrin reaction, Ryndon Smith reaction, iodine reaction, anisaldehyde sulfuric acid reaction, and phosphomolybdic acidperchloric acid reaction, and a negative BTB and p-anisidine reaction. Note, as the compound I-j was obtained in only a small amount, the exact structure thereof was not determined. The structures and physico-chemical properties of the compounds I-a to I-i are shown in Table 3.

Table 3

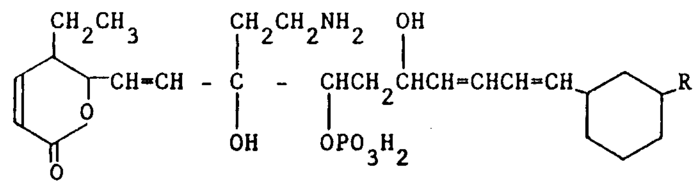

| Compound | R | Molecular formula | Rf value* | IR spectrum ($\nu cm^{-1}$) | NMR spectrum (in $CD_3OD$, $\delta$ ppm) |
|---|---|---|---|---|---|
| I-a | H | $C_{25}H_{40}O_8NP$ | 0.45 | 2968, 2934, 1730, 1604, 1137 | 7.09(1H,dd,J=10.0,5.0), 6.26(1H,m), 6.23 (1H,m), 6.08(1H,dd,J=15.5,6.5), 6.02(1H, dd,J=10.0,1.5), 5.92(1H,dd,J=15.5,1.0), 5.4(1H,m), 5.31(1H,m), 5.10(1H,ddd, J=6.5,5.5,1.0), 4.95(1H,m), 4.27(1H,ddd, J=10.0,10.0,3.0), 3.04(2H,m), 2.56(1H,m), 2.46(1H,m), 2.2(1H,ddd,J=7.0), 1.88(1H, ddd,J=7.0), 1.55-1.76(6H,m), 1.44-1.55 (2H,m), 1.24-1.42(3H,m), 1.04-1.24(3H,m), 0.95(3H,dd,J=7.5) |
| I-b | 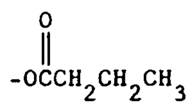 | $C_{29}H_{46}O_{10}NP$ | 0.48 | 2968, 2935, 1728, 1603, 1093 | 0.93(3H,t,J=6.2), 0.94(3H,t,J=7.0), 1.04 (1H,m), 1.14(1H,m), 1.29(1H,m), 1.37-1.55 (3H,m), 1.55-1.67(4H,m), 1.72(1H,m), 1.78-1.89(2H,m), 1.89-2.01(2H,m), 2.20 (1H,m), 2.25(2H,t,J=7.2), 2.50-2.70(2H,m), 2.94-3.13(2H,m), 4.29(1H,dt,J=2.0,J=10.0), 4.70(1H,m), 4.95(1H,d,J=10.0), 5.10(1H,dd, J=5.0,J=6.8), 5.31(1H,m), 5.46(1H,m), 5.95(1H,d,J=15.9), 6.02(1H,dd, J=10.0,J=1.5), 6.05(1H,dd, J=6.8,J=15.9), 6.22-6.33(2H,m), 7.08(1H,dd, J=5.5,J=10.2) |

Table 3 (continued)

| Compound | R | Molecular formula | Rf value* | IR spectrum ($\nu cm^{-1}$) | NMR spectrum (in $CD_3OD$, $\delta$ ppm) |
|---|---|---|---|---|---|
| I-c | 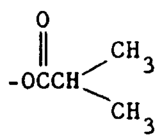 | $C_{29}H_{46}O_{10}NP$ | 0.48 | 2968, 2935 1728, 1603, 1093 | 0.94(3H,t,J=7.0), 1.04(1H,m), 1.12(6H,d,J=7.0), 1.14(1H,m), 1.29(1H,m), 1.37-1.55 (3H,m), 1.55-1.67(2H,m), 1.72(1H,m), 1.78-1.89(2H,m), 1.89-2.01(2H,m), 2.20(1H,m), 2.49(1H,sep,J=7.0), 2.51-2.69(2H,m), 2.94-3.13(2H,m), 4.29(1H,dt,J=2.0,J=10.0), 4.70(1H,m), 4.95(1H,d,J=10.0), 5.10(1H,dd, J=5.0,J=6.8), 5.31(1H,m), 5.46(1H,m), 5.95(1H,d,J=15.9), 6.02(1H,dd,J=10.0,J=1.5), 6.05(1H,dd,J=6.8,J=15.9), 6.22-6.33(2H,m), 7.08(1H,dd,J=5.5,J=10.2) |
| I-d | 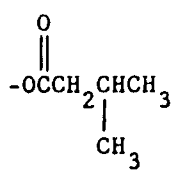 | $C_{30}H_{48}O_{10}NP$ | 0.49 | 3374, 2872, 1726, 1383, 1294, 1188 | 0.94(6H,d,J=6.7), 0.95(3H,t,J=8.0), 1.05 (1H,m), 1.15(1H,q,J=11.8), 1.28(1H,m), 1.40-1.54(3H,m), 1.57-1.67(2H,m), 1.73 (1H,m) 1.78-1.89(2H,m), 1.91-2.01(2H,m), 2.04(1H,m), 2.15(2H,d,J=7.2), 2.19(1H,m), 2.56(1H,m), 2.63(1H,m), 3.00(1H,m), 3.08 (1H,m), 4.30(1H,dt,J=2.5,J=10.1), 4.73 (1H,m), 4.94(1H,m), 5.10(1H,dd,J=6.6, J=4.7), 5.31(1H,m), 5.46(1H,m), 5.95(1H,d, J=15.6), 6.02(1H,dd,J=10.0,J=1.2), 6.05 (1H,dd,J=15.6,J=6.6), 6.24-6.32(2H,m), 7.08(1H,dd,J=10.0,J=5.0) |

Table 3 (continued)

| Compound | R | Molecular formula | Rf value* | IR spectrum ($\nu cm^{-1}$) | NMR spectrum (in $CD_3OD$, δ ppm) |
|---|---|---|---|---|---|
| I-e | 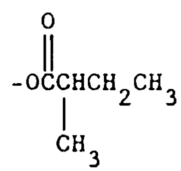 | $C_{30}H_{48}O_{10}NP$ | 0.49 | 3374, 2872, 1726, 1383, 1294, 1188 | 0.89(3H,t,J=7.5), 0.95(3H,t,J=8.0), 1.05(1H,m), 1.10(3H,d,J=6.9), 1.15(1H,q,J=11.8), 1.28(1H,m), 1.40-1.54(4H,m), 1.57-1.67(3H,m), 1.73(1H,m), 1.78-1.89(2H,m), 1.91-2.01(2H,m), 2.19(1H,m), 2.32(1H,m), 2.56(1H,m), 2.63(1H,m), 3.00(1H,m), 3.08(1H,m), 4.30(1H,dt,J=2.5,J=10.1), 4.73(1H,m), 4.94(1H,m), 5.10(1H,dd,J=10.0,J=1.2), 6.05(1H,dd,J=15.6,J=6.6), 6.24-6.32(2H,m), 7.08(1H,dd,J=10.0,J=5.0) |
| I-f | 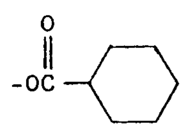 | $C_{32}H_{50}O_{10}NP$ | 0.50 | 3455, 2932, 1722, 1451, 1383, 1318, 1246, 1173 | 0.95(3H,t,J=7.5), 1.05(1H,m), 1.14(1H,q,J=11.8), 1.19-1.36(4H,m), 1.36-1.54(5H,m), 1.58-1.67(3H,m), 1.68-1.76(3H,m), 1.79-1.87(4H,m), 1.90-1.98(2H,m), 2.20(1H,m), 2.26(1H,m), 2.55(1H,m), 2.62(1H,m), 3.01(1H,m), 3.07(1H,m), 4.29(1H,dt,J=2.4,J=10.1), 4.69(1H,m), 4.94(1H,m), 5.10(1H,dd,J=4.7,J=6.7), 5.31(1H,m), 5.46(1H,m), 5.95(1H,d,J=15.6), 6.02(1H,dd,J=1.3,J=9.8), 6.06(1H,dd,J=6.7,J=15.6), 6.23-6.32(2H,m), 7.08(1H,dd,J=5.1,J=9.8) |

Table 3 (continued)

| Compound | R | Molecular formula | Rf value* | IR spectrum ($\nu cm^{-1}$) | NMR spectrum (in $CD_3OD$, δ ppm) |
|---|---|---|---|---|---|
| I-g | 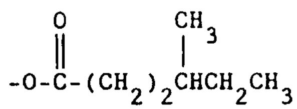 | $C_{32}H_{52}O_{10}NP$ | 0.50 | 3358, 2963, 1726, 1711, 1464, 1450, 1383, 1253 | 0.84-0.91(6H,m), 0.95(3H,t,J=7.4), 0.98-1.23(3H,m), 1.23-1.55(7H,m), 1.55-1.77(4H,m), 1.77-1.89(2H,m), 1.89-2.02(2H,m), 2.15-2.35(3H,m), 2.51-2.68(2H,m), 2.95-3.13(2H,m), 4.28(1H,dt,J=2.0,J=10.0), 4.71(1H,m), 4.93(1H,m), 5.09(1H,dd,J=5.2,J=6.4), 5.31(1H,m), 5.45(1H,m), 5.94(1H,d,J=15.6), 6.01(1H,d,J=9.8), 6.05(1H,dd,J=6.4,J=15.6), 6.22-6.33(2H,m), 7.08(1H,dd,J=9.8,J=5.1) |
| I-h | 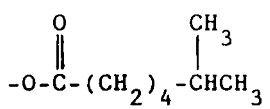 | $C_{33}H_{54}O_{10}NP$ | 0.52 | 2935, 2865, 1730, 1240, 1174, 1057 | 0.88(6H,d,J=6.7), 0.95(3H,t,J=7.5), 1.04(1H,m), 1.10-1.22(3H,m), 1.23-1.36(3H,m), 1.39-1.67(8H,m), 1.72(1H,t,J=12.2), 1.79-1.89(2H,m), 1.91-2.03(2H,m), 2.17-2.25(1H,m), 2.27(2H,t,J=7.4), 2.55(1H,m), 2.62(1H,m), 3.01(1H,m), 3.08(1H,m), 4.30(1H,t,J=9.4), 4.72(1H,m), 4.94(1H,m), 5.10(1H,m), 5.31(1H,m), 5.46(1H,m), 5.95(1H,d,J=15.6), 6.02(1H,dd,J=9.8,J=1.2), 6.05(1H,dd,J=15.6,J=6.6), 6.23-6.32(2H,m), 7.08(1H,dd,J=9.8,J=5.1) |

Table 3 (continued)

| Compound | R | Molecular formula | Rf value* | IR spectrum ($\nu cm^{-1}$) | NMR spectrum (in $CD_3OD$, $\delta$ ppm) |
| --- | --- | --- | --- | --- | --- |
| I-i | $-O-\overset{\overset{O}{\|}}{C}-(CH_2)_6CH_3$ | $C_{33}H_{54}O_{10}NP$ | 0.52 | 2930, 2867, 1730, 1250, 1170, 1057 | 0.90(3H,t,J=7.0), 0.95(3H,t,J=7.5), 1.05 (1H,m), 1.15(1H,q,J=11.8), 1.19-1.37(8H, m), 1.38-1.54(4H,m), 1.54-1.76(5H,m), 1.79-1.89(2H,m), 1.91-2.01(2H,m), 2.17-2.25(1H,m), 2.27(2H,t,J=7.3), 2.55(1H,m), 2.62(1H,m), 3.01(1H,m), 3.08(1H,m), 4.30 (1H,dt,J=9.2,J=1.0), 4.72(1H,m), 4.94(1H, m), 5.10(1H,m), 5.31(1H,m), 5.46(1H,m), 5.95(1H,d,J=15.5), 6.02(1H,dd,J=9.8,J=1.1), 6.05(1H,dd,J=15.5,J=6.6), 6.23-6.33 (2H,m), 7.08(1H,dd,J=9.8,J=5.1) |

* Silica gel thin layer chromatography (Merck HPTLC Silica F254; developing solvent = chroloform/methanol/water 6:4:1; detected by bioautography using *Aspergillus oryzae*, anisaldehyde-sulfuric acid reaction and ninhydrin reaction.

## Test for anti-fungal activity

The disease suppressive activity of the present compounds I-a, I-c (and I-b), I-d (and I-e), I-f, I-g, I-h, and I-i against the gray mold was tested by the following procedure. A solution of the present compound

having a predetermined concentration was coated,with an absorbent cotton block, on the cotyledon of a cucumber seedling seven days after seeding. One day after the coating, an agar disk having a diameter of 5 mm containing Botrytis cinerea was put on the coated cotyledon, the plant was incubated at 20°C for three days, and the disease suppresive activity of the tested compound was observed. The results are shown in Table 4. In Table 4, Botrytis cinerea RR-4 is a strain resistant to antimicrobial agents (multi-resistant strain), isolated from a diseased seedling of egg plant, and Botrytis cinerea S-9 is a strain sensitive to antimicrobial agents, isolated from a diseased petal of orange blossom. Note, the extent of the disease suppressive effect is expressed by symbols + (no lesion), ± (lesion having a diameter of less than 10 mm formed), and - (lesion having a diameter of not less than 10 mm formed).

Table 4

| Disease-suppressive effect on Botrytis cinerea | | | |
|---|---|---|---|
| Test compound | Concentration (ppm) | Botrytis cinerea S-9 | Botrytis cinerea RR-4 |
| I-a | 125<br>32 | +<br>- | +<br>- |
| I-b and I-c (1:2) | 125<br>32 | +<br>± | +<br>± |
| I-d and I-e (3:2) | 125<br>32<br>8 | +<br>±<br>- | +<br>±<br>- |
| I-f | 32<br>8 | +<br>+ | +<br>+ |
| I-g | 32<br>8 | +<br>+ | +<br>+ |
| I-h | 32<br>8 | +<br>- | +<br>- |
| I-i | 32<br>8 | +<br>- | +<br>- |
| Procymidone | 125<br>32 | +<br>- | -<br>- |
| No treatment | | - | - |

**Claims**

**Claims for the following Contracting States : AT, DE, FR, GB, IT, SE**

**1.** A compound which is

(i) a 2-pyranone compound of formula

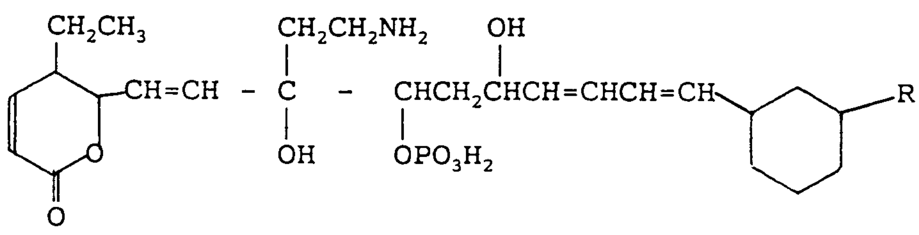

wherein R represents a hydrogen atom, or a linear or branched alkylcarbonyloxy or cycloalkylcarbonyloxy group having 3 to 10 carbon atoms; or
(ii) a salt thereof.

2. A compound according to claim 1 in which R is butyryloxy, isobutyryloxy, isovaleryloxy, 2-methylbutyryloxy, cyclohexylcarbonyloxy, 4-methylhexanoyloxy, 6-methylheptanoyloxy, or octanoyloxy.

3. A process for production of a compound according to claim 1 or claim 2, comprising culturing a microorganism, of the strain Streptomyces platensis SAM-0654 (FERM BP-1668) to produce the compound, and recovering the produced compound from the culture.

4. A fungicidal composition comprising an effective amount of a compound according to claim 1 or claim 2.

5. A composition according to claim 4 comprising a carrier which is
a solid powder or granular carrier, and in which the compound constitutes from 0.5 to 50wt% of the composition, or
a liquid carrier, and in which the compound constitutes from 5 to 90wt% of the composition which is for dilution to 10 to 1000ppm of compound.

6. The microorganism Streptomyces platensis SAM-0654 (FERM BP-1668).

7. A method of treating plants to destroy the pathogenic fungus Botrytis cinerea, comprising applying to the plants a compound according to claim 1 or claim 2.

8. A method according to claim 7 in which the compound is applied at from 10 to 300g per 10 ares.

**Claims for the following Contracting State : ES**

1. A process comprising the production of a compound which is
(i) a 2-pyranone compound of the formula

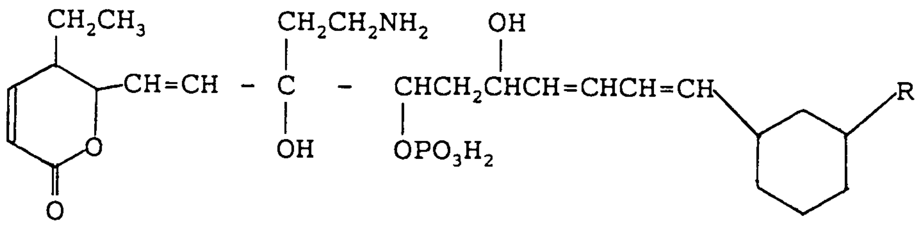

wherein R represents a hydrogen atom, or a linear or branched alkylcarbonyloxy or cycloalkylcarbonyloxy group having 3 to 10 carbon atoms; or
(ii) a salt thereof,
comprising culturing a microorganism of the strain Streptomyces platensis SAM-0654 (FERM BP-1668) to produce the compound, and recovering the produced compound from the culture.

2. A process according to claim 1 in which R is butyryloxy, isobutyryloxy, isovaleryloxy, 2-methylbutyryloxy, cyclohexylcarbonyloxy, 4-methylhexanoyloxy, 6-methylheptanoyloxy, or octanoyloxy.

3. A process according to claim 1 or claim 2, further comprising mixing the compound with a carrier to form a fungicidal composition.

4. A process according to claim 3 in which
the carrier is a powder or granular solid carrier, and the compound constitutes from 0.5 to 50wt% of the composition, or
the carrier is a liquid carrier, and the compound constitutes from 5 to 90wt% of the composition, which is for dilution to 10 to 1000ppm of compound.

5. A method of treating plants to destroy the pathogenic fungus Botrytis cinerea, comprising applying to the plants a compound as defined in claim 1 or claim 2.

6. A method according to claim 5 in which the compound is applied at from 10 to 300g per 10 ares.

**Patentansprüche**
**Patentansprüche für folgende Vertragsstaaten : AT, DE, FR, GB, IT, SE**

1. Eine Verbindung, die
(i) eine 2-Pyranonverbindung der Formel

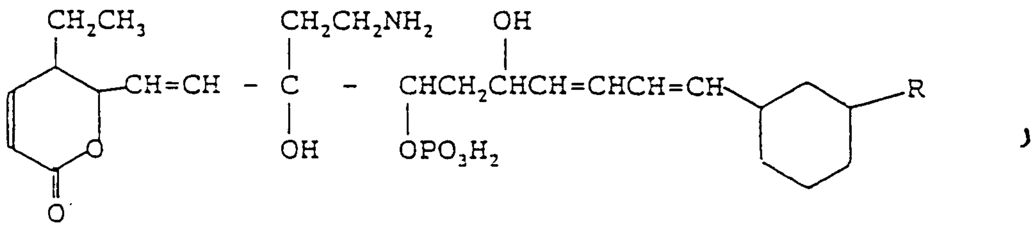

in der R ein Wasserstoffatom oder eine lineare oder verzweigte Alkylcarbonyloxy- oder Cycloalkylcarbonyloxygruppe mit 3 bis 10 Kohlenstoffatomen ist, oder
(ii) deren Salz ist.

2. Verbindung nach Anspruch 1, in welcher R Butyryloxy, Isobutyryloxy, Isovaleryloxy, 2-Methylbutyryloxy, Cyclohexylcarbonyloxy, 4-Methylhexanoyloxy, 6-Methylheptanoyloxy oder Octanoyloxy ist.

3. Verfahren zum Herstellen einer Verbindung nach Anspruch 1 oder Anspruch 2 durch Kultivieren eines Mikroorganismus des Stammes Streptomyces platensis SAM-0654 (FERM BP-1668), um die Verbindung zu erzeugen, und Wiedergewinnen der erzeugten Verbindung aus der Kultur.

4. Fungizidzusammensetzung enthaltend eine wirksame Menge einer Verbindung nach Anspruch 1 oder Anspruch 2.

5. Zusammensetzung nach Anspruch 4 mit einem Träger, der
ein Festpulver- oder granulärer Träger ist und in dem die Verbindung von 0,5 bis 50 Gew.-% der Zusammensetzung darstellt, oder
ein flüssiger Träger ist, in welchem die Verbindung von 5 bis 90 Gew.-% der Zusammensetzung darstellt, die für die Verdünnung auf 10 bis 1000 ppm der Verbindung ist.

6. Mikroorganismus Streptomyces platensis SAM-0654 (FERM BP-1668).

7. Verfahren zum Behandeln von Pflanzen zum Zerstören des pathogenen Pilzes Botrytis cinerea durch Aufbringen einer Verbindung nach Anspruch 1 oder Anspruch 2 auf die Pflanzen.

8. Verfahren nach Anspruch 7, in welchem die Verbindung von 10 bis 300 g pro 10 Ar aufgebracht wird.

**Patentansprüche für folgenden Vertragsstaat : ES**

1. Verfahren zur Herstellung einer Verbindung, die
   (i) eine 2-Pyranonverbindung der Formel

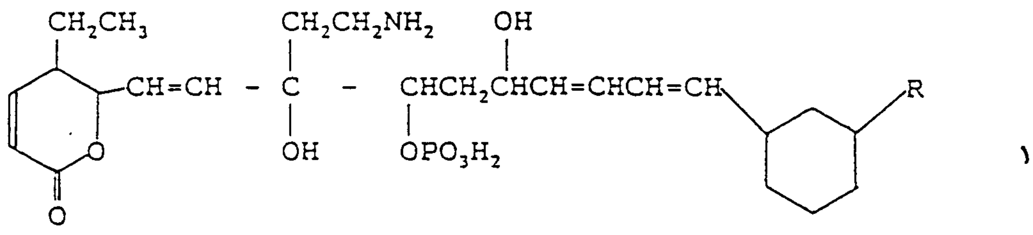

   in der R ein Wasserstoffatom oder eine lineare oder verzweigte Alkylcarbonyloxy- oder Cycloalkylcarbonyloxygruppe mit 3 bis 10 Kohlenstoffatomen ist, oder
   (ii) deren Salz ist,
   durch Kultivieren eines Mikroorganismus des Stammes Streptomyces platensis SAM-0654 (FERM BP-1668), um die Verbindung zu erzeugen, und Wiedergewinnen der erzeugten Verbindung aus der Kultur.

2. Verfahren nach Anspruch 1, in welcher R Butyryloxy, Isobutyryloxy, Isovaleryloxy, 2-Methylbutyryloxy, Cyclohexylcarbonyloxy, 4-Methylhexanoyloxy, 6-Methylheptanoyloxy oder Octanoyloxy ist.

3. Verfahren nach Anspruch 1 oder Anspruch 2, welches weiter das Mischen der Verbindung mit einem Träger unter Bildung einer Fungizidzusammensetzung umfaßt.

4. Verfahren nach Anspruch 3, in welchem
   der Träger ein Pulver- oder granulärer Festträger ist und die Verbindung von 0,5 bis 50 Gew.-% der Zusammensetzung darstellt, oder
   der Träger ein flüssiger Träger ist und die Verbindung von 5 bis 90 Gew.-% der Zusammensetzung darstellt, welcher zur Verdünnung auf 10 bis 1000 ppm der Verbindung ist.

5. Verfahren zur Behandlung von Pflanzen zum Zerstören des pathogenen Pilzes Botrytis cinerea durch Aufbringen einer Verbindung nach Anspruch 1 oder Anspruch 2 auf die Pflanzen.

6. Verfahren nach Anspruch 5, in welchem die Verbindung von 10 bis 300 g pro 10 Ar aufgebracht wird.

**Revendications**
**Revendications pour les Etats contractants suivants : AT, DE, FR, GB, IT, SE**

1. Un dérivé qui est:
   (i) un dérivé de 2-pyranone représenté par la formule:

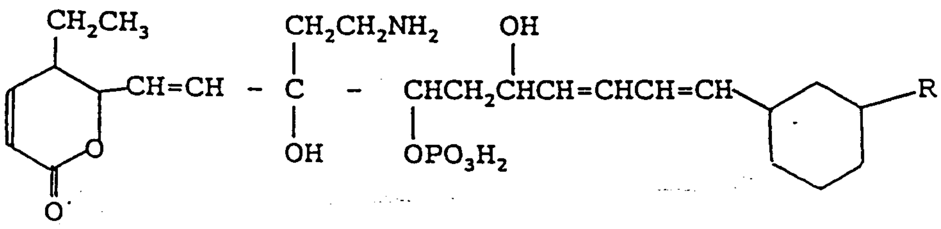

   dans laquelle:
   R représente un atome d'hydrogène, ou un groupe alkylcarbonyloxy linéaire ou ramifié ou cycloalkylcarbonyloxy, renfermant de 3 à 10 atomes de carbone; ou
   (ii) un de ses sels.

**2.** Un dérivé selon la revendication 1, dans lequel R représente un groupe butyryloxy, isobutyryloxy, isovaléryloxy, 2-méthylburyryloxy, cyclohexylcarbonyloxy, 4-méthylhexanoyloxy, 6-méthyl-heptanoyloxy, ou octanoyloxy.

**3.** Un procédé de préparation d'un dérivé selon la revendication 1 ou 2, comprenant la mise en culture d'un micro-organisme de la souche de Streptomyces platensis SAM-O654 (FERM BP-1668) pour produire le dérivé, et la récupération du dérivé obtenu à partir de la culture.

**4.** Une Composition fongicide contenant une quantité efficace d'un dérivé selon la revendication 1 ou 2.

**5.** Une composition selon la revendication 4, comprenant un support qui est:
- une poudre solide ou un support granulaire, et dans lequel le dérivé constitue de 0,5 à 50% en poids de la composition, ou
- un support liquide et dans lequel le dérivé constitue de 5 à 90% en poids de la composition destinée à une dilution à 10 à 1000 ppm du dérivé.

**6.** Le micro-organisme Streptomyces platensis SAM-0654 (FERM BP-1668).

**7.** Un procédé de traitement des Végétaux pour détruire le champignon pathogène Botrytis cinerea, comprenant l'application sur les végétaux d'un dérivé selon la revendication 1 ou 2.

**8.** Un procédé selon la revendication 7, dis lequel le dérivé est appliqué en une quantité de 10 à 300 g pour 10 ares.

**Revendication pour l'Etat contractant suivant : ES**

**1.** Un procédé comprenant la production d'un dérivé qui est:
(i) un dérivé de 2-pyranone représenté par la formule:

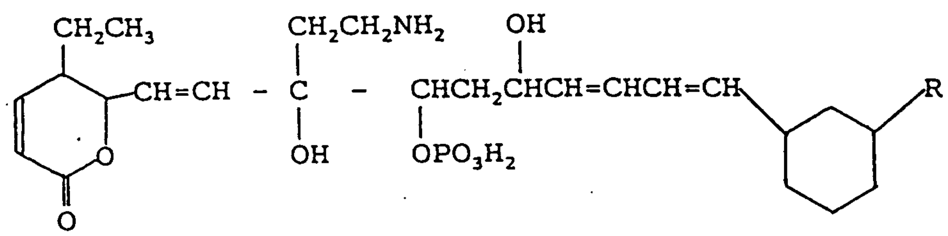

dans laquelle:
R représente un atome d'hydrogène, ou un groupe alkylcarbonyloxy linéaire ou ramifié ou cycloalkylcarbonyloxy, renfermant de 3 à 10 atomes de carbone; ou
(ii) un de leurs sels,
comprenant la culture d'un micro-organisme de la souche Streptomyces platensis SAM-0654 (FERM BP-1668) pour produire le dérivé, et la récupération du dérivé ainsi obtenu à partir de la culture.

**2.** Un procédé selon la revendication 1, dans lequel R représente un groupe butyryloxy, isobutyryloxy, isovaléryloxy, 2-méthylbutyryloxy, cyclohexylcarbonyloxy, 4-méthylhexanoyloxy, 6-méthyl-heptanoyloxy ou octanoyloxy.

**3.** Un procédé selon la revendication 1 ou 2, comprenant en outre le mélange du dérivé avec un support pour former une composition fongicide.

**4.** Un procédé selon la revendication 3, dans lequel:
- le support est une poudre ou un support solide granulaire, et le dérivé constitue de 0,5 à 50% en poids de la composition, ou
- le support est un support liquide et le dérivé constitue de 5 à 90% en poids de la composition qui est destinée à une dilution à 10 à 1000 ppm du dérivé.

**5.** Un procédé de traitement des végétaux pour détruire le champignon pathogène Botrytis cinerea, comprenant l'application sur les végétaux d'un dérivé tel que défini dans la revendication 1 ou 2.

**6.** Un procédé selon la revendication 5, dans lequel le dérivé est appliqué en une quantité de 10 à 300g pour 10 ares.

Fig. 1

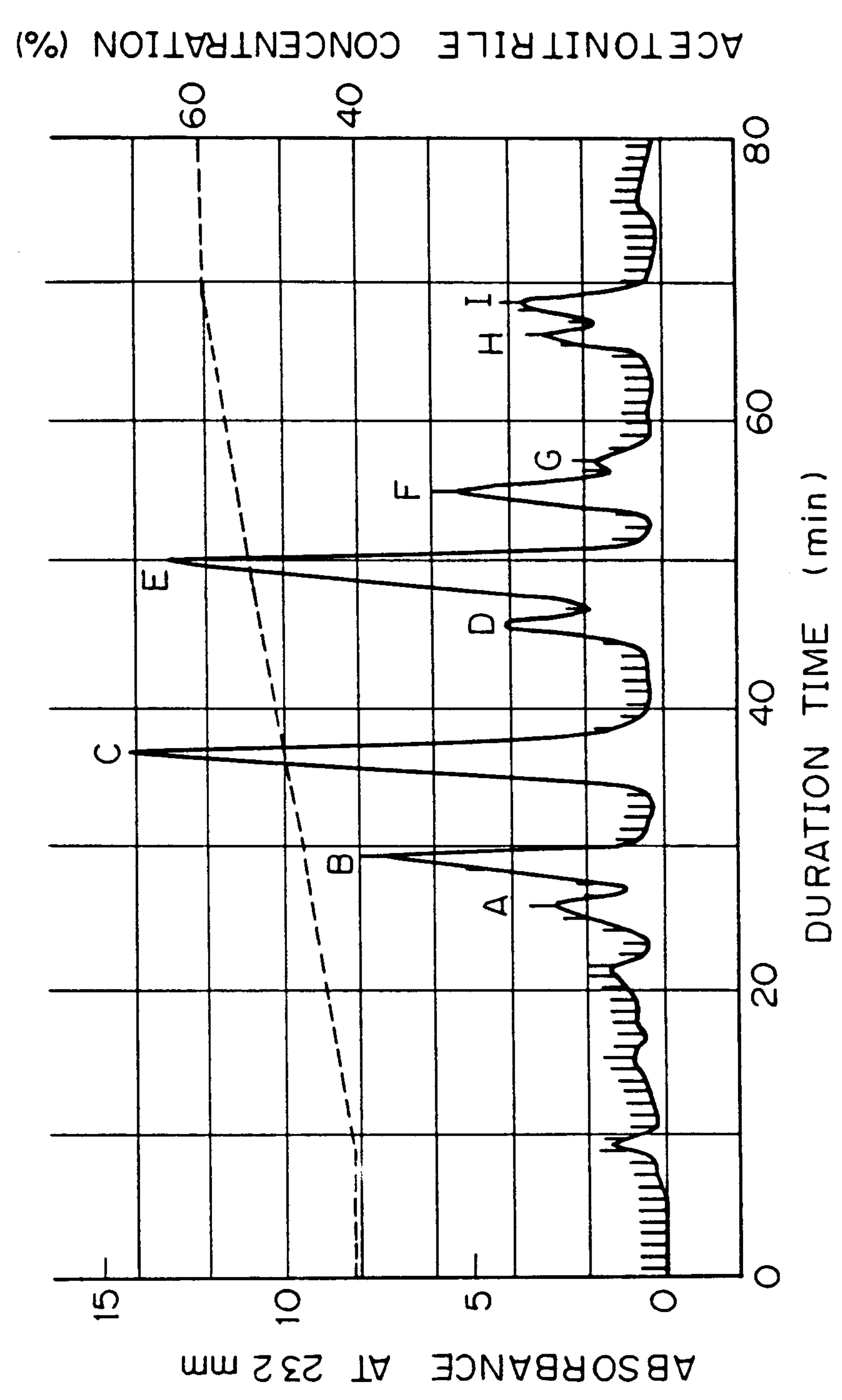
ACETONITRILE CONCENTRATION (%)
60
40
ABSORBANCE AT 232 mm
15
10
5
0
DURATION TIME (min)
0
20
40
60
80
A
B
C
D
E
F
G
H
I